# EUROPEAN PATENT SPECIFICATION

(11) **EP 3 315 121 B1**
(45) Date of publication and mention of the grant of the patent: **03.03.2021**
(21) Application number: 17195299.7
(22) Date of filing: 06.10.2017
(51) Int. Cl.: A61K 9/08, A61K 47/18, A61K 47/02, A61K 47/36, A61K 9/107, A61K 47/44

(54) **PHARMACEUTICAL COMPOSITIONS**
PHARMAZEUTISCHE ZUSAMMENSETZUNGEN
COMPOSITIONS PHARMACEUTIQUES

(30) Priority: 27.10.2016 GB 201618175; 18.01.2017 GB 201700831
(43) Date of publication of application: 02.05.2018
(73) Proprietor: Warneford Healthcare Limited, Weybridge KT13 0TT (GB)
(72) Inventor: FRASER, Scott, Newcastle-Upon-Tyne, Tyne & Wear NE3 4EX (GB); PRIESTLEY, Graham, Weybridge, KT13 0TT (GB)
(74) Representative: Swindell & Pearson Limited

(56) References cited:
- WO-A1-2008/071528
- WO-A1-2013/065866
- WO-A2-2010/141648
- US-A1- 2013 090 308
- US-A1- 2014 221 309
- US-B2- 9 119 827

## Description

The present invention is concerned with ophthalmic pharmaceutical compositions, and in particular ophthalmic pharmaceutical compositions that are suitable for treatment of dry eye, and especially treatment thereof during sleep.

Dry eye syndrome is a multi-factorial disease of the tears and their production resulting in ocular surface pathology associated with symptoms of discomfort, visual disturbance, and tear film instability with potential damage to the ocular surface (exposed eye ball, sclera, conjunctival sac, cornea). Dry eye is associated with increased osmolality of the tear film and inflammation of the ocular surface due to improper secretion production of various secretory glands in the eye lids including lacrimal glands.

Dry eye is an increasingly common problem for patients. As stated above, it is a multi-factorial disease of the tears and often results in a vicious cycle of drying, damage, irritation, inflammation and further damage to the ocular surface. Optimal relief from dry eye requires treatment of all parts of this cycle. A truly effective drop to treat dry eye needs to replace the aqueous component of the tears, supplement the lipid layer, stabilise the mucin layer, osmoprotect the corneal epithelium and to hydrate its cells as well as enhancing their healing.

Dry eye usually affects both the eyes. Tears, a watery fluid, are mostly produced by lacrimal glands. Without tears, good vision is not possible. Dry eye is a common ophthalmological disorder increasingly affecting the population older than 40 years. Dry eye is greater in the Hispanic and Asian populations than in the Caucasian population, and an estimated 30 million people suffer from this condition worldwide. It is very prevalent in the Middle East population due to dry desert extreme weather conditions.

During the day a sufferer is able to administer multiple drops to seek to treat or alleviate the symptoms of dry eye, but this is self-evidently not true during the night, when a drop that will have prolonged contact with the ocular surface is needed. A benefit of nocturnal treatment is that it is an opportunity to manipulate the environment between the eyelid and the ocular surface of the eye so as to promote healing and repair during this prolonged contact time, that is when the sufferer is asleep and their eye is closed. Preferably this drop will be free from toxic preservatives and will be delivered from a device that prevents bacterial contamination of the content of the device.

The present invention provides an ophthalmic pharmaceutical composition that is particularly suited for treatment of dry eye during sleep. According to a first aspect of the present invention, the present invention therefore, there is provided an ophthalmic pharmaceutical composition which comprises castor oil, levocarnitine, and a pharmaceutical vehicle suitable for topical application to an eye of a patient. According to a second aspect of the present invention, the present invention, there is provided an ophthalmic pharmaceutical composition which comprises levocarnitine, and a pharmaceutical vehicle suitable for topical application to an eye of a patient.

Suitable castor oil to be used in a composition according to the first aspect of the present invention can comprise polyethoxylated castor oils, such as those classified as PEG-2 to PEG-200 castor oils, as well as those classified as PEG-5 to PEG-200 hydrogenated castor oils. Such polyethoxylated castor oils include those manufactured by Rhone-Poulenc (Cranbury, New Jersey) under the Alkamuls brand, those manufactured by BASF (Parsippany, New Jersey) under the Cremophor brand, and those manufactured by Nikko Chemical Co., Ltd. (Tokyo, Japan) under the Nikkol brand. Particularly suitable polyethoxylated castor oils are those classified as PEG-15 to PEG-50 castor oils, and more preferred are PEG-30 to PEG-35 castor oils. Typically, therefore, polyethoxylated castor oils known as Cremophor EL or Alkamuls EL-620 can be used.

Typically castor oil is included in a composition according to the present invention in an amount of less than about 0.35 w/w % of the composition, more typically in an amount of about 0.20 w/w % to about 0.30 w/w %, such as about 0.25 w/w %. Alternatively, the castor oil is included in a composition according to the present invention in an amount of less than about 0.35 w/w % of the composition, more typically in an amount of about 0.005 w/w % to about 0.10 w/w %, such as about 0.01 w/w%.

The lipid layer of the tear films is essential to reduce aqueous tear evaporation and prolongs the wetting ability of the tears. Loss or reduction of this lipid layer results in drying of the ocular surface and damage to the corneal epithelium. This can occur even when aqueous tear production is normal.

This lipid layer can be effectively replaced by eye drops. The inclusion of castor oil in an ophthalmic pharmaceutical composition according to the first aspect of the present invention is well tolerated, and effectively replaces the lipid layer. Still further, the presence of such castor oil persists in the tear film for well over four hours after administration of a composition according to the present invention, making the composition particularly suitable for use during sleep of a patient.

The castor oil will preferably be combined with an emulsifier in an ophthalmic pharmaceutical composition according to the first aspect of the present invention, thus allowing the castor oil to be suspended and distributed throughout the composition.

Typically the emulsifier may be present in an amount of less than about 0.60 w/w % of the composition, more typically in an amount of about 0.20 w/w % to about 0.50 w/w %. Alternatively, the emulsifier may be present in an amount of less than about 2.00 w/w % of the composition, more typically in an amount of about 1.50 w/w % to about 0.50 w/w %, typically about 1.0 w/w %. A particularly suitable emulsifier in either embodiment comprises polysorbate 80.

Hyperosmolarity of the tear film is a common occurrence in dry eye disease. It is one of components of the vicious cycle of continued ocular surface damage. Hypotonic tear substances counteract this but their effect is too short lived. Research has indicated that some molecules can act as osmoprotectors, protecting the ocular surface from the hyperosmolar damage and breaking this part of the dry eye damage cycle. There are a number of substances that can do this such as glycerol and erythritol. However, a composition according to the present invention comprises an osmoprotector with a duration of action suitable for an overnight treatment, namely levocarnitine. Typically, levocarnitine achieves an osmolality of a composition according to the present invention at a level at, or near, 210-320 millimoles per kilogram.

Typically levocarnitine may be present in an amount of less than about 0.60 w/w % of the composition, more typically in an amount of about 0.10 w/w % to about 0.50 w/w %, such as about 0.25 w/w %.

According to a particularly preferred embodiment of the present invention there is provided an ophthalmic pharmaceutical composition which comprises about 0.20 w/w % to about 0.30 w/w % of castor oil, about 0.10 w/w % to about 0.50 w/w % of levocarnitine and a pharmaceutical vehicle suitable for topical application to an eye of a patient.

According to a particularly preferred embodiment of the present invention there is provided an ophthalmic pharmaceutical composition which comprises about 0.10 w/w % to about 0.50 w/w % of levocarnitine and a pharmaceutical vehicle suitable for topical application to an eye of a patient.

The pharmaceutical vehicle suitable for topical application to an eye of a patient may be water, purified water or artificial tears. Preferably, the vehicle is purified water.

Typically, an ophthalmic pharmaceutical composition according to the present invention further comprises an ocular lubricant. The lubricant may comprise a cellulose derivative such as carboxymethyl cellulose, but more preferably the ocular lubricant comprises hyaluronic acid or a pharmaceutically acceptable salt thereof. Particularly suitable are alkali metal salts of hyaluronic acid, such as sodium hyaluronate.

Sodium hyaluronate has unique physical and chemical characteristics that combine the viscous properties of a liquid with the elastic properties of a solid - depending on the shear stress applied. This means that when used as an ocular lubricant, it dramatically increases the stability of the tear film and maximizes tear film residence time protecting the corneal epithelium from drying, restoring the tear film and so giving prolonged comfort.

Sodium hyaluronate has a number of properties that make it the ideal ocular lubricant and it is a substance that can mimic and replace each layer of the normal tear film:
(i) It mimics and stabilizes the mucin layer of the tears
(ii) Sodium hyaluronate, because of its structure, retains a large amount of water meaning better and more prolonged wetting of the ocular surface. This essentially means that the aqueous layer of the tears is replaced naturally and for a prolonged period of time.
(iii) Sodium hyaluronate offers a high degree of protection to the corneal epithelial cells combining mechanical protection with hydration of the cells. It provides an optimal environment (as in the normal eye) where the epithelium can thrive and cells are able to repair themselves if damaged.
(iv) Osmolality - Sodium hyaluronate has been shown to reduce tear osmolality and has a synergistic and prolonged effect when used with other osmoprotectors, in the case of the present invention levocarnitine.

Typically sodium hyaluronate may be present in an amount of less than about 0.50 w/w % of an ophthalmic pharmaceutical composition according to the present invention, more typically in an amount of about 0.35 w/w % to about 0.45 w/w %, such as an amount of about 0.40 w/w %. The sodium hyaluronate may be of any suitable molecular weight for use in an ophthalmic pharmaceutical composition. The molecular weight and the Intrinsic viscosity of sodium hyaluronate are related. A preferred Intrinsic viscosity of the sodium hyaluronate for use in compositions according to the present invention is about 1.8 m³ / Kg.

Sodium hyaluronate as present in an ophthalmic pharmaceutical composition according to the present invention persists in the eye up to seven times longer than standard eye drops. Sodium hyaluronate typically has a tear persistency of at least six hours as measured when the eye is open and blinking normally. During sleep when the eye is closed there is very little evaporation from the ocular surface and no drainage through the lacrimal system, which means that in a composition according to the present invention when administered prior to sleep and as such when subsequently present in a patient's eye during sleep, sodium hyaluronate has a tear persistency of at least nine hours, or more preferably at least twelve hours.

According to a particularly preferred embodiment of the present invention there is provided an ophthalmic pharmaceutical composition which comprises castor oil, levocarnitine, sodium hyaluronate and a pharmaceutical vehicle suitable for topical application to an eye of a patient. More specifically, the composition comprises about 0.20 w/w % to about 0.30 w/w % of castor oil, about 0.10 w/w % to about 0.50 w/w % of levocarnitine, about 0.35 w/w % to about 0.45 w/w % of sodium hyaluronate and a pharmaceutical vehicle suitable for topical application to an eye of a patient. Preferably the Intrinsic viscosity of the sodium is 1.8 m³ / Kg.

According to a particularly preferred embodiment of the present invention there is provided an ophthalmic pharmaceutical composition which comprises castor oil, levocarnitine, sodium hyaluronate and a pharmaceutical vehicle suitable for topical application to an eye of a patient. More specifically, the composition comprises about 0.001 w/w % to about 0.30 w/w % of castor oil, typically 0.01 w/w % of castor oil, about 0.10 w/w % to about 0.50 w/w % of levocarnitine, typically 0.25 w/w % of levocarnitine, about 0.35 w/w % to about 0.45 w/w % of sodium hyaluronate, typically 0.4 w/w %, and a pharmaceutical vehicle suitable for topical application to an eye of a patient. Preferably the Intrinsic viscosity of the sodium is 1.8 m³ / Kg.

According to a particularly preferred embodiment of the present invention there is provided an ophthalmic pharmaceutical composition which comprises levocarnitine, sodium hyaluronate and a pharmaceutical vehicle suitable for topical application to an eye of a patient. More specifically, the composition comprises about 0.10 w/w % to about 0.50 w/w % of levocarnitine, typically 0.25 w/w % of levocarnitine, about 0.35 w/w % to about 0.45 w/w % of sodium hyaluronate, typically 0.4 w/w % of Sodium hyaluronate, and a pharmaceutical vehicle suitable for topical application to an eye of a patient. Preferably the Intrinsic viscosity of the sodium is 1.8 m³ / Kg.

Typically, a composition according to the present invention further comprises a source of zinc, and preferably this is provided in the form of zinc chloride. Typically the source of zinc, such as zinc chloride, is present in an amount of about 0.0001 w/w % to about 0.15 w/w %, and is more preferably present in an amount of about 0.0025 w/w %.

Zinc is an essential trace element in the cornea. Deficiency causes a number of corneal abnormalities. Zinc supplementation of tears has been shown to enhance corneal epithelium healing. It not only allows repair of damaged epithelial cells but also promotes cell division. The abnormal environment created by dry eyes results in the loss of areas of epithelium - one of the major reasons for ocular discomfort. Rapid cell division as is promoted by the presence of zinc is essential to quickly close these exposed areas making the patient more comfortable and reducing inflammation.

A further advantage associated with the use of a source of zinc in an ophthalmic pharmaceutical composition according to the present invention, is that zinc binds to hyaluronic acid and / or a pharmaceutically acceptable salt thereof. This means that the potency of the zinc is enhanced in an ophthalmic pharmaceutical composition according to the present invention but without any increase in concentration. As such, the amount of zinc can be lowered without loss of effect in an ophthalmic pharmaceutical composition according to the present invention. Typically, therefore a source of zinc is included in an ophthalmic pharmaceutical composition according to the present invention at a sub-therapeutic amount compared to the typical level of zinc in other commercial ophthalmic compositions.

According to a particularly preferred embodiment of the present invention there is provided an ophthalmic pharmaceutical composition which comprises castor oil, levocarnitine, sodium hyaluronate, zinc chloride and a pharmaceutical vehicle suitable for topical application to an eye of a patient. More specifically, the composition comprises about 0.20 w/w % to about 0.30 w/w % of castor oil, about 0.10 w/w % to about 0.50 w/w % of levocarnitine, typically 0.25 w/w % of levocarnitine, about 0.35 w/w % to about 0.45 w/w % of sodium hyaluronate, typically 0.4 w/w % of Sodium hyaluronate, about 0.0001 w/w % to about 0.15 w/w % of zinc chloride, typically 0.0025 w/w % of zinc chloride, and a pharmaceutical vehicle suitable for topical application to an eye of a patient. Preferably the Intrinsic viscosity of the sodium is 1.8 m³ / Kg.

According to a particularly preferred embodiment of the present invention there is provided an ophthalmic pharmaceutical composition which comprises castor oil, levocarnitine, sodium hyaluronate, zinc chloride and a pharmaceutical vehicle suitable for topical application to an eye of a patient. More specifically, the composition comprises about 0.001 w/w % to about 0.30 w/w % of castor oil, typically 0.01 w/w % of castor oil, about 0.10 w/w % to about 0.50 w/w % of levocarnitine, typically 0.25 w/w % of levocarnitine, about 0.35 w/w % to about 0.45 w/w % of sodium hyaluronate, typically 0.4 w/w % of Sodium hyaluronate, about 0.0001 w/w % to about 0.15 w/w % of zinc chloride, typically 0.0025 w/w % of zinc chloride, and a pharmaceutical vehicle suitable for topical application to an eye of a patient. Preferably the Intrinsic viscosity of the sodium is 1.8 m³ / Kg.

According to a particularly preferred embodiment of the present invention there is provided an ophthalmic pharmaceutical composition which comprises levocarnitine, sodium hyaluronate, zinc chloride and a pharmaceutical vehicle suitable for topical application to an eye of a patient. More specifically, the composition comprises about 0.10 w/w % to about 0.50 w/w % of levocarnitine, typically 0.25 w/w % of levocarnitine, about 0.35 w/w % to about 0.45 w/w % of sodium hyaluronate, typically 0.4 w/w % of Sodium hyaluronate, about 0.0001 w/w % to about 0.15 w/w % of zinc chloride, typically 0.0025 w/w % of zinc chloride, and a pharmaceutical vehicle suitable for topical application to an eye of a patient. Preferably the Intrinsic viscosity of the sodium is 1.8 m³ / Kg.

Preservatives are generally used in eye drop bottles to prevent bacterial contamination of the drops. However, such preservatives can be toxic to the corneal epithelium and disrupt the tear film. This is especially so when drops are used frequently and over a prolonged time, such as for the treatment of dry eye disease. It is therefore preferred that the compositions of the present invention are substantially free of any preservatives, especially any chloride containing preservatives. Particularly, the composition is free of benzalkonium chloride.

As used herein, the phrase "substantially free of" as it refers to a preservative means that an ophthalmic pharmaceutical composition according to the present invention is either entirely devoid of a preservative or includes only a nominal amount of a preservative. Preferably, an ophthalmic pharmaceutical composition according to the present invention is entirely devoid of any preservatives.

According to a particularly preferred embodiment of the present invention there is provided an ophthalmic pharmaceutical composition which comprises castor oil, levocarnitine, sodium hyaluronate, zinc chloride and a pharmaceutical vehicle suitable for topical application to an eye of a patient, which composition is substantially free of any preservatives. More specifically, the composition comprises about 0.20 w/w % to about 0.30 w/w % of castor oil, about 0.10 w/w % to about 0.50 w/w % of levocarnitine, about 0.35 w/w % to about 0.45 w/w % of sodium hyaluronate, about 0.0001 w/w % to about 0.15 w/w % of zinc chloride and a pharmaceutical vehicle suitable for topical application to an eye of a patient, which composition is substantially free of any preservatives. Preferably the Intrinsic viscosity of the sodium is 1.8 m³ / Kg.

According to a particularly preferred embodiment of the present invention there is provided an ophthalmic pharmaceutical composition which comprises castor oil, levocarnitine, sodium hyaluronate, zinc chloride and a pharmaceutical vehicle suitable for topical application to an eye of a patient, which composition is substantially free of any preservatives. More specifically, the composition comprises about 0.001 w/w % to about 0.30 w/w % of castor oil, typically 0.01 w/w % of castor oil, about 0.10 w/w % to about 0.50 w/w % of levocarnitine, typically 0.25 w/w % of levocarnitine, about 0.35 w/w % to about 0.45 w/w % of sodium hyaluronate, typically 0.4 w/w % of Sodium hyaluronate, about 0.0001 w/w % to about 0.15 w/w % of zinc chloride, typically 0.0025 w/w % of zinc chloride, and a pharmaceutical vehicle suitable for topical application to an eye of a patient, which composition is substantially free of any preservatives. Preferably the Intrinsic viscosity of the sodium is 1.8 m³ / Kg.

According to a particularly preferred embodiment of the present invention there is provided an ophthalmic pharmaceutical composition which comprises levocarnitine, sodium hyaluronate, zinc chloride and a pharmaceutical vehicle suitable for topical application to an eye of a patient, which composition is substantially free of any preservatives. More specifically, the composition comprises about 0.10 w/w % to about 0.50 w/w % of levocarnitine, typically 0.25 w/w % of levocarnitine, about 0.35 w/w % to about 0.45 w/w % of sodium hyaluronate, typically 0.4 w/w % of Sodium hyaluronate, about 0.0001 w/w % to about 0.15 w/w % of zinc chloride, typically 0.0025 w/w % of zinc chloride, and a pharmaceutical vehicle suitable for topical application to an eye of a patient, which composition is substantially free of any preservatives. Preferably the Intrinsic viscosity of the sodium is 1.8 m³ / Kg.

In a particularly preferred embodiment of the present invention, there is provided an ophthalmic pharmaceutical composition which comprises about 0.25% w/w % of castor oil with Polysorbate 80 as an emulsifier, about 0.25 w/w % of levocarnitine, about 0.40 w/w % of sodium hyaluronate, about 0.0025 w/w % of zinc chloride, with the remaining w/w % balance to 100% comprising purified water, which composition is substantially free of any preservatives. Preferably the Intrinsic viscosity of the sodium is 1.8 m³ / Kg.

In a particularly preferred embodiment of the present invention, there is provided an ophthalmic pharmaceutical composition which comprises about 0.01% w/w % of castor oil with Polysorbate 80 as an emulsifier, about 0.25 w/w % of levocarnitine, about 0.40 w/w % of sodium hyaluronate, about 0.0025 w/w % of zinc chloride, with the remaining w/w % balance to 100% comprising purified water, which composition is substantially free of any preservatives. Preferably the Intrinsic viscosity of the sodium is 1.8 m³ / Kg.

In a particularly preferred embodiment of the present invention, there is provided an ophthalmic pharmaceutical composition which comprises about 0.25 w/w % of levocarnitine, about 0.40 w/w % of sodium hyaluronate, about 0.0025 w/w % of zinc chloride, with the remaining w/w % balance to 100% comprising purified water, which composition is substantially free of any preservatives. Preferably the Intrinsic viscosity of the sodium is 1.8 m³ / Kg.

An ophthalmic pharmaceutical composition according to the present invention is a liquid, cream or gel, advantageously exhibiting prolonged tear persistency. Such a composition is a significant physical and chemical protector of the ocular surface and has constituents that create optimal conditions for damaged epithelial cells to repair and regenerate. This liquid, cream or gel is therefore unique in its ability to both protect and heal, breaking the vicious cycle of dry eye by treating each part of the cycle. Most preferably the liquid, cream or gel will be applied to the ocular surface of the eye shortly before a patient goes to sleep.

There is thus provided by the present invention an ophthalmic pharmaceutical composition which comprises castor oil, levocarnitine, and a pharmaceutical vehicle suitable for topical application to an eye of a patient, for use in the treatment of dry eye.

Still more preferred is an ophthalmic pharmaceutical composition which comprises castor oil, levocarnitine, and a pharmaceutical vehicle suitable for topical application to an eye of a patient, for use in the treatment of dry eye and wherein said composition is to be administered prior to sleep of the patient. Typically, the composition is administered within 60 minutes prior to sleep of a patient, typically within 30 minutes prior to sleep of a patient, or typically within 15 minutes prior to sleep of a patient.

There is further provided by the present invention a kit comprising an ophthalmic pharmaceutical composition which comprises castor oil, levocarnitine, and a pharmaceutical vehicle suitable for topical application to an eye of a patient, together with instructions to administer the composition so as to treat dry eye of the patient.

Still further according to the present invention there is provided a kit comprising an ophthalmic pharmaceutical composition which comprises castor oil, levocarnitine, and a pharmaceutical vehicle suitable for topical application to an eye of a patient, together with instructions to administer prior to sleep of the patient so as to treat dry eye of the patient during sleep. Typically, the instructions advise administration of the composition within 60 minutes prior to sleep of a patient, typically within 30 minutes prior to sleep of a patient, or typically within 15 minutes prior to sleep of a patient.

There is still further provided by the present invention a method of treating dry eye in a patient, which method comprises topically administering to an eye of the patient an ophthalmic pharmaceutical composition which comprises castor oil, levocarnitine, and a pharmaceutical vehicle suitable for topical application to an eye of a patient. Preferably, the method comprises administering the composition prior to sleep of the patient, so as to treat dry eye of the patient during sleep. Typically, administration is within 60 minutes prior to sleep of a patient, typically within 30 minutes prior to sleep of a patient, or typically within 15 minutes prior to sleep of a patient. A method according to the present invention thus advantageously provides nocturnal treatment of dry eye.

The present invention further comprises a suitable dispensing device for administration of an ophthalmic pharmaceutical composition to an eye of a patient, which device contains an ophthalmic pharmaceutical composition which comprises castor oil, levocarnitine, and a pharmaceutical vehicle suitable for topical application to an eye of a patient. The device needs to be able to deliver precise amounts of such a composition to the eye of a patient and must also allow for ease of handling by the patient for administration to one or both eyes sequentially. Still further, the device should prevent direct handling of the contained composition by the patient, and thus obviate bacterial contamination of the composition.

A preferred dispensing device is an eyedrop dispenser available under the trade mark Aptar. This allows preservative free drops to be dispensed from an eye drop bottle. This is done using a unique cap on the bottle - allowing the instillation of eye drops but preventing bacterial ingress into the bottle.

Further preferred features of a composition to be used in a method, therapeutic use, kit and / or dispensing device according to the present invention are as hereinbefore described in greater detail. However, in a most preferred embodiment of the present invention, there is provided a dispensing device available under the trade mark Aptar, which contains an ophthalmic pharmaceutical composition which comprises about 0.25 w/w % of castor oil together with polysorbate 80 as an emulsifier, about 0.25 w/w % of levocarnitine, about 0.40 w/w % of sodium hyaluronate, about 0.0025 w/w % of zinc chloride, with the remaining w/w % balance to 100 % comprising purified water, and which composition is substantially free of preservatives.

Further preferred features of a composition to be used in a method, therapeutic use, kit and / or dispensing device according to the present invention are as hereinbefore described in greater detail. However, in a most preferred embodiment of the present invention, there is provided a dispensing device available under the trade mark Aptar, which contains an ophthalmic pharmaceutical composition which comprises about 0.01 w/w % of castor oil together with polysorbate 80 as an emulsifier, about 0.25 w/w % of levocarnitine, about 0.40 w/w % of sodium hyaluronate with an Intrinsic viscosity of about 1.8 m³ / Kg., about 0.0025 w/w % of zinc chloride, with the remaining w/w % balance to 100 % comprising purified water, and which composition is substantially free of preservatives.

Further preferred features of a composition to be used in a method, therapeutic use, kit and / or dispensing device according to the present invention are as hereinbefore described in greater detail. However, in a most preferred embodiment of the present invention, there is provided a dispensing device available under the trade mark Aptar, which contains an ophthalmic pharmaceutical composition which comprises about 0.25 w/w % of levocarnitine, about 0.40 w/w % of sodium hyaluronate, about 0.0025 w/w % of zinc chloride, with the remaining w/w % balance to 100 % comprising purified water, and which composition is substantially free of preservatives.

Further preferred features of a composition to be used in a method, therapeutic use, kit and / or dispensing device according to the present invention are as hereinbefore described in greater detail. However, in a most preferred embodiment of the present invention, there is provided a dispensing device available under the trade mark Aptar, which contains an ophthalmic pharmaceutical composition which comprises about 0.25 w/w % of levocarnitine, about 0.40 w/w % of sodium hyaluronate with an Intrinsic viscosity of about 1.8 m³ / Kg., about 0.0025 w/w % of zinc chloride, with the remaining w/w % balance to 100 % comprising purified water, and which composition is substantially free of preservatives.

The present invention will now be further illustrated by the following Examples, which do not limit the scope of the claims in any way.

### Example 1:

| **Component** | **w/w %** |
|---|---|
| Castor oil together with polysorbate 80 as an emulsifier | 0.25 |
| Levocarnitine | 0.25 |
| Sodium hyaluronate | 0.40 |
| Zinc chloride | 0.0025 |
| Purified water | q.s. to 100 |

### Example 2:

| **Component** | **w/w %** |
|---|---|
| Levocarnitine | 0.25 |
| Sodium hyaluronate | 0.40 |
| Zinc chloride | 0.0025 |
| Purified water | q.s. to 100 |

### Example 3:

| **Component** | **w/w %** |
|---|---|
| Castor oil | 0.01 |
| Polysorbate 80 as an emulsifier | 1.0 |
| Levocarnitine | 0.25 |
| Sodium hyaluronate with an Intrinsic viscosity of about 1.8 m³ / Kg | 0.4 |
| Zinc chloride | 0.0025 |
| Disodium phosphate dodecahydrate as a pH buffer | 0.3 |
| Sodium dihydrogen phosphate monohydrate as a pH buffer | 0.066 |
| Sodium chloride as an osmotic stabiliser | 0.8 |
| Purified water | q.s. to 100 |

### Example 4:

| **Component** | **w/w %** |
|---|---|
| Levocarnitine | 0.25 |
| Sodium hyaluronate with an Intrinsic viscosity of about 1.8 m³ / Kg | 0.40 |
| Zinc chloride | 0.0025 |
| Sodium chloride as an osmotic stabiliser | 0.5 |
| Boric Acid as a pH buffer | 0.8 |
| Borax as a pH buffer | 0.05 |
| Purified water | q.s. to 100 |

## Claims

1. An ophthalmic pharmaceutical composition which comprises about 0.005 w/w % to about 0.30 w/w % of castor oil, about 0.10 w/w % to about 0.50 w/w % of levocarnitine, about 0.35 w/w % to about 0.45 w/w % of sodium hyaluronate, from about 0.0001 w/w % to about 0.15 w/w % of the composition of zinc chloride, and a pharmaceutical vehicle suitable for topical application to an eye of a patient.

2. A composition according to claim 1 which is substantially free of any preservatives.

3. An ophthalmic pharmaceutical composition which comprises castor oil, levocarnitine, sodium hyaluronate, zinc chloride and a pharmaceutical vehicle suitable for topical application to an eye of a patient, which composition is substantially free of any preservatives.

4. An ophthalmic pharmaceutical composition which comprises about 0.25% w/w %, of castor oil with Polysorbate 80 as an emulsifier, about 0.25 w/w % of levocarnitine, about 0.40 w/w % of sodium hyaluronate, about 0.0025 w/w % of zinc chloride, with the remaining w/w % balance to 100 % comprising purified water, which composition is substantially free of any preservatives.

5. An ophthalmic pharmaceutical composition which comprises about 0.01 w/w % of castor oil together with Polysorbate 80 as an emulsifier, about 0.25 w/w % of levocarnitine, about 0.40 w/w % of sodium hyaluronate, about 0.0025 w/w % of zinc chloride, with the remaining w/w % balance to 100 % comprising purified water, and which composition is substantially free of preservatives.

6. A composition according to any of claims 1 to 5, for use in the treatment of dry eye and wherein said composition is administered prior to sleep of the patient or less than 60 minutes prior to sleep of a patient, or within 15 to 60 minutes prior to sleep of a patient, or 15 to 30 minutes prior to sleep of a patient for the nocturnal treatment of dry eye.

7. A kit comprising a composition according to any of claims 1 to 6, together with instructions to administer the composition prior to sleep of a patient or less than 60 minutes prior to sleep of a patient, or within 15 to 60 minutes prior to sleep of a patient, or 15 to 30 minutes prior to sleep of a patient so as to treat dry eye of the patient during sleep.

8. A dispensing device for administration of an ophthalmic pharmaceutical composition to an eye of a patient, which device contains a composition according to any of claims 1 to 6.

9. A device according to claim 8 together with instructions to administer the composition prior to sleep of the patient or less than 60 minutes prior to sleep of a patient, or within 15 to 60 minutes prior to sleep of a patient, or 15 to 30 minutes prior to sleep of a patient so as to treat dry eye of the patient during sleep.

## Patentansprüche

1. Ophthalmische pharmazeutische Zusammensetzung, die etwa 0,005 Gew.-% bis etwa 0,30 Gew.-% Rizinusöl, etwa 0,10 Gew.-% bis etwa 0,50 Gew .-% Levocarnitin, etwa 0,35 Gew.-% bis etwa 0,45 Gew.-% Natriumhyaluronat, etwa 0,0001 Gew.-% bis etwa 0,15 Gew.-% der Zusammensetzung Zinkchlorid, und ein pharmazeutisches Mittel umfasst, das zur topischen Anwendung an einem Auge eines Patienten geeignet ist.

2. Zusammensetzung nach Anspruch 1, die im Wesentlichen frei von jeglichen Konservierungsmitteln ist.

3. Ophthalmische pharmazeutische Zusammensetzung, die Rizinusöl, Levocarnitin, Natriumhyaluronat, Zinkchlorid und ein pharmazeutisches Mittel umfasst, das zur topischen Anwendung an einem Auge eines Patienten geeignet ist, wobei die Zusammensetzung im Wesentlichen frei von jeglichen Konservierungsmitteln ist.

4. Ophthalmische pharmazeutische Zusammensetzung, die etwa 0,25 Gew.-% Rizinusöl mit Polysorbat 80 als Emulgator, etwa 0,25 Gew.-% Levocarnitin, etwa 0,40 Gew.-% Natriumhyaluronat, etwa 0,0025 Gew.-% Zinkchlorid umfasst, wobei die verbleibende Gew.-% Differenz bis 100% gereinigtes Wasser umfasst, wobei die Zusammensetzung im Wesentlichen frei von Konservierungsmitteln ist.

5. Ophthalmische pharmazeutische Zusammensetzung, die etwa 0,01 Gew.-% Rizinusöl zusammen mit Polysorbat 80 als Emulgator, etwa 0,25 Gew.-% Levocarnitin, etwa 0,40 Gew.-% Natriumhyaluronat, etwa 0,0025 Gew.-% Zinkchlorid umfasst, wobei die verbleibende Gew.-% Differenz bis 100% gereinigtes Wasser umfasst, und wobei die Zusammensetzung im Wesentlichen frei von Konservierungsmitteln ist.

6. Zusammensetzung nach einem der Ansprüche 1 bis 5 zur Verwendung bei der Behandlung trockenen Auges und wobei die Zusammensetzung vor dem Schlafen des Patienten oder weniger als 60 Minuten vor dem Schlafen eines Patienten oder innerhalb von 15 bis 60 Minuten vor dem Schlafen eines Patienten oder 15 bis 30 Minuten vor dem Schlafen eines Patienten zur nächtlichen Behandlung trockenen Auges verabreicht wird.

7. Kit umfassend eine Zusammensetzung nach einem der Ansprüche 1 bis 6, zusammen mit Anweisungen zur Verabreichung der Zusammensetzung vor dem Schlafen eines Patienten oder weniger als 60 Minuten vor dem Schlafen eines Patienten oder innerhalb von 15 bis 60 Minuten vor dem Schlafen eines Patienten oder 15 bis 30 Minuten vor dem Schlafen eines Patienten, um ein trockenes Auge des Patienten während des Schlafes zu behandeln.

8. Abgabevorrichtung zur Verabreichung einer ophthalmischen pharmazeutischen Zusammensetzung an ein Auge eines Patienten, wobei die Vorrichtung eine Zusammensetzung gemäß einem der Ansprüche 1 bis 6 enthält.

9. Vorrichtung nach Anspruch 8 zusammen mit Anweisungen zum Verabreichen der Zusammensetzung vor dem Schlafen des Patienten oder weniger als 60 Minuten vor dem Schlafen eines Patienten oder innerhalb von 15 bis 60 Minuten vor dem Schlafen eines Patienten oder 15 bis 30 Minuten vor dem Schlafen des Patienten, um ein trockenes Auge des Patienten während des Schlafes zu behandeln.

## Revendications

1. Composition pharmaceutique ophtalmique qui comprend environ 0,005% p / p à environ 0,30% p / p d'huile de ricin, environ 0,10% p / p à environ 0,50% p / p de lévocarnitine, environ 0,35% p / p à environ 0,45% p / p de hyaluronate de sodium, environ 0,0001% p / p à environ 0,15% p / p de la composition de chlorure de zinc, et un véhicule pharmaceutique approprié pour une application topique sur un œil d'un patient.

2. Composition selon la revendication 1 laquelle est sensiblement exempte de tout conservateur.

3. Composition pharmaceutique ophtalmique qui comprend de l'huile de ricin, de la lévocarnitine, du hyaluronate de sodium, du chlorure de zinc et un véhicule pharmaceutique approprié pour une application topique sur un œil d'un patient, laquelle composition est sensiblement exempte de tout conservateur.

4. Composition pharmaceutique ophtalmique qui comprend environ 0,25% p / p d'huile de ricin avec du Polysorbate 80 comme émulsifiant, environ 0,25% p / p de lévocarnitine, environ 0,40% p / p de hyaluronate de sodium, environ 0,0025% p / p de chlorure de zinc, le % p / p restant restant jusqu'aux 100% comprenant de l'eau purifiée, laquelle composition est sensiblement exempte de tout conservateur.

5. Composition pharmaceutique ophtalmique qui comprend environ 0,01% p / p d'huile de ricin avec du Polysorbate 80 comme émulsifiant, environ 0,25% p / p de lévocarnitine, environ 0,40% p / p de hyaluronate de sodium, environ 0,0025% p / p de chlorure de zinc, le % p / p restant jusqu'aux 100% comprenant de l'eau purifiée, et laquelle composition est sensiblement exempte de conservateurs.

6. Composition selon l'une quelconque des revendications 1 à 5, à utiliser dans le traitement de la sécheresse oculaire et dans laquelle ladite composition est administrée avant le sommeil du patient ou moins de 60 minutes avant le sommeil d'un patient, ou dans les 15 à 60 minutes avant le sommeil d'un patient, ou 15 à 30 minutes avant le sommeil d'un patient pour le traitement nocturne de la sécheresse oculaire.

7. Kit comprenant une composition selon l'une quelconque des revendications 1 à 6, ainsi que des instructions pour administrer la composition avant le sommeil d'un patient ou moins de 60 minutes avant le sommeil d'un patient, ou dans les 15 à 60 minutes avant le sommeil d'un patient, ou 15 à 30 minutes avant le sommeil d'un patient afin de traiter la sécheresse oculaire du patient pendant le sommeil.

8. Dispositif de distribution pour l'administration d'une composition pharmaceutique ophtalmique à un œil d'un patient, lequel dispositif contient une composition selon l'une quelconque des revendications 1 à 6.

9. Dispositif selon la revendication 8 avec des instructions pour administrer la composition avant le sommeil du patient ou moins de 60 minutes avant le sommeil d'un patient, ou dans les 15 à 60 minutes avant le sommeil d'un patient, ou 15 à 30 minutes avant le sommeil d'un patient afin de traiter la sécheresse oculaire du patient pendant son sommeil.
